# EUROPEAN PATENT APPLICATION

(11) **EP 4 008 282 A1**
(43) Date of publication of application: **08.06.2022**
(21) Application number: 21172811.8
(22) Date of filing: 07.05.2021
(51) Int. Cl.: A61B 17/86

(54) **BONE SCREW**

(30) Priority: 04.12.2020 TW 109142790
(71) Applicant: Industrial Technology Research Institute, Hsinchu 31040 (TW)
(72) Inventor: SHEN, Hsin-Hsin, 310401 Hsinchu (TW); TSAI, Pei-I, 310 Hsinchu County (TW); YANG, Kuo-Yi, 300 Hsinchu City (TW); JANG, Fang-Jie, 204 Keelung City (TW); CHEN, An-Li, 736 Tainan City (TW); LIN, Shih-Ping, 805 Kaohsiung City (TW); HUANG, Chih-Chieh, 350 Miaoli County (TW); WEN, Yi-Hung, 300 Hsinchu City (TW); FAN, Wei-Lun, 350 Miaoli County (TW); HUANG, Shin-I, 300 Hsinchu City (TW)
(74) Representative: dompatent von Kreisler Selting Werner - Partnerschaft von Patent- und Rechtsanwälten mbB

(57) **Abstract**

A bone screw includes an external screw thread, an internal supporter structure and a porous layer. The internal supporter structure is inside the external screw thread. The internal supporter structure includes a screw thread supporter or a polygonal wall supporter. The porous layer is on a surface of the internal supporter structure. Therefore, the bone screw has an increased strength and also provides postoperative healing effect.

## Description

### TECHNICAL FIELD

The disclosure relates in general to a bone screw, and more particularly to a bone screw having an internal supporter structure.

### BACKGROUND

When one's issues, such as bones, tendons or ligaments, are damaged to a certain degree, doctors may decide that one's damaged part may need a surgery. Normally, the surgery is to move one's tissues to the pre-damage position and fix the tissues using surgical sutures and bone implants such as bone screws. Then, one's damaged part will be recovered through one's self-repair ability. Or, some man-made tissues can be fixed inside the body using sutures and bone implants to assist or replace the damaged tissues. Most of the tissues, such as shoulders, knees and adjacent tissues, have mobility requirement. The bone implants need to have excellent biological compatibility and suitable mechanical function and possess physical and chemical properties, such that the bone implants despite being used under a long duration of stress cycle still can match the needs of organism.

The bone implants can be formed of metallic or nonmetallic materials. Metallic materials, such as stainless steel or titanium alloy, have better mechanical properties and can sustain the load received by the bone implants. However, metallic bone implants have a hardness much larger than a hardness of the bone and may easily generate a stress shielding effect over the long run, causing discomfort to the surgical part, making the surrounding tissues of the implanted part loose and delaying bone healing. To the worse, osseointegration may fail and the affected part may need another surgery, which increases further surgical risk and medical costs.

### SUMMARY

The disclosure is directed to a bone screw.

According to one embodiment, a bone screw is provided. The bone screw includes an external screw thread, an internal supporter structure and a porous layer. The internal supporter structure is disposed inside a middle part of the external screw thread. The internal supporter structure includes one of a screw thread supporter and a polygonal wall supporter. The porous layer is disposed on a surface of the internal supporter structure.

According to another embodiment, a bone screw is provided. The bone screw includes an external screw thread and an internal supporter structure. The internal supporter structure is disposed inside a middle part of the external screw thread. The internal supporter structure is a polygonal wall supporter with several.

The above and other aspects of the invention will become better understood with regard to the following detailed description of the preferred but non-limiting embodiment (s). The following description is made with reference to the accompanying drawings.

### BRIEF DESCRIPTION OF THE DRAWINGS

FIG. 1 is a 3D appearance diagram of a bone screw according to an embodiment.
FIG. 2 is a longitudinal cross-sectional view of a bone screw according to an embodiment.
FIG. 3 is a top view of a top pin portion a bone screw according to an embodiment.
FIG. 4 is a 3D appearance diagram of a bone screw according to another embodiment.
FIG. 5 is a schematic diagram of a porous layer according to an embodiment.
FIG. 6 is a schematic diagram of a porous layer according to another embodiment.
FIG. 7 a schematic diagram of a porous layer according to yet another embodiment.
FIG. 8 is a 3D appearance diagram of a bone screw according to another embodiment.
FIG. 9 is a longitudinal cross-sectional view of a bone screw according to another embodiment.
FIG. 10 is a top view of a top pin portion of a bone screw according to another embodiment.
FIG. 11 is a lateral cross-sectional view of a portion of a polygonal wall supporter without apertures.
FIG. 12 is a lateral cross-sectional view of a portion of a polygonal wall supporter with apertures.
FIG. 13 is a 3D appearance diagram of a bone screw according to yet another embodiment.
FIG. 14 is a 3D appearance diagram of a bone screw according to yet more another embodiment.
FIG. 15 is a lateral cross-sectional view of a portion of a polygonal wall supporter with apertures.

In the following detailed description, for purposes of explanation, numerous specific details are set forth in order to provide a thorough understanding of the disclosed embodiments. It will be apparent, however, that one or more embodiments may be practiced without these specific details. In other instances, well-known structures and devices are schematically shown in order to simplify the drawing.

### DETAILED DESCRIPTION

A number of embodiments are disclosed below for explanatory purpose. It should be noted that although the present disclosure does not illustrate all possible embodiments, other embodiments not disclosed in the present disclosure are still applicable. Moreover, the dimension scales used in the accompanying drawings are not based on actual proportion of the product. Therefore, the specification and drawings are for explaining and describing the embodiment only, not for limiting the scope of protection of the present disclosure. Furthermore, descriptions of the embodiments, such as detailed structures, manufacturing procedures and materials, are for exemplification purpose only, not for limiting the scope of protection of the present disclosure. Suitable modifications or changes can be made to the structures and procedures of the embodiments to meet actual needs without breaching the spirit of the present disclosure. Designations common to the accompanying drawings are used to indicate identical or similar elements.

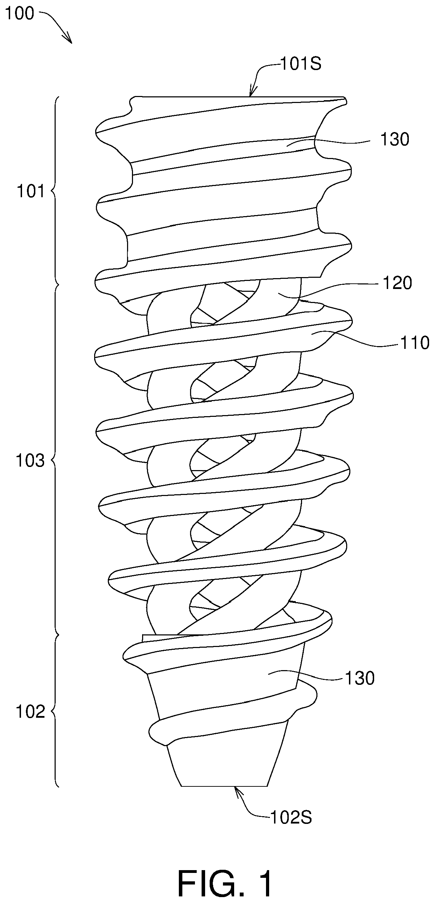

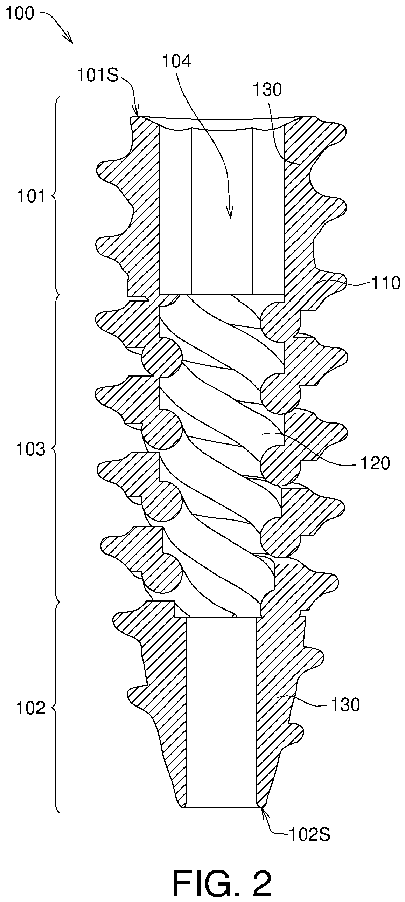

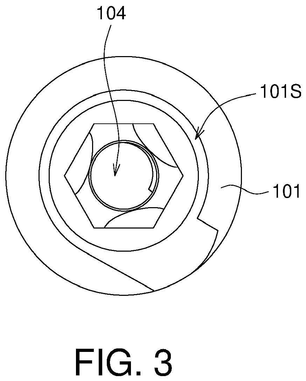

A bone screw 100 in an embodiment is disclosed with reference to FIG. 1, FIG. 2 and FIG. 3. FIG. 1 is a 3D appearance diagram of the bone screw 100. FIG. 2 is a longitudinal cross-sectional view of the bone screw 100. FIG. 3 is a top view of a top pin portion 101 the bone screw 100.

The bone screw 100 includes an external screw thread 110 and an internal supporter structure. In the present embodiment, the internal supporter structure includes a screw thread supporter 120. The screw thread supporter 120 is inside a middle part of the external screw thread 110. A spiral direction of the external screw thread 110 can be identical to or different from a spiral direction of the screw thread supporter 120. The screw thread supporter 120 is not limited to a three-wire screw thread, and can comprise supporter structure of a one-wire screw thread, a two-wire screw thread, a four-wire screw thread, or a screw thread of other amounts of wires. A diameter of the screw thread supporter 120 may be 0.8 mm to 1.2 mm, for example, but the present disclosure is not limited thereto.

Parts of the bone screw 100 may include the top pin portion 101, a bottom pin portion 102 and a middle pin portion 103, for example. The middle pin portion 103 is between the top pin portion 101 and the bottom pin portion 102. The top pin portion 101, the bottom pin portion 102 and the middle pin portion 103 include the external screw thread 110. Otherwise, the external screw thread 110 connects the top pin portion 101, the bottom pin portion 102 and the middle pin portion 103. Each of the top pin portion 101 and the bottom pin portion 102 includes an outer wall layer 130 connecting with the top and the bottom of the external screw thread 110. The middle pin portion 103 includes the screw thread supporter 120 and a part of the external screw thread 110 (such as the middle part of the external screw thread 110). The bone screw 100 includes a middle hole 104 passing through the interior of the bone screw 100. The middle hole 104 can be inside the screw thread supporter 120. The middle hole 104 can be extended to the bottom bone screw surface 102S of the bottom pin portion 102 from the top bone screw surface 101S of the top pin portion 101 along an axial direction. The hollowed middle hole 104 can be used for surgical guidance and micro-wound surgery.

In an embodiment, materials of the external screw thread 110, the outer wall layer 130 and the screw thread supporter 120 can have a non-porous structure. The non-porous structure refers to a solid structure or material without pores connected with each other. The material of the internal supporter structure (the screw thread supporter 120) has a solid structure (that is, non-porous structure), which provides the bone screw 100 with sufficient structural strength and hardness, reduces the probability of rupture during the implantation or use process and increases the lifespan of the bone screw 100. In comparison to the bone screw of the comparison example without having an internal supporter structure, the bone screw 100 of the present embodiment having the screw thread supporter 120 having the material being a solid structure (that is, a non-porous structure) as an internal supporter structure can have larger torsional strength and pull-out strength. The external screw thread 110 can be used for increasing the stability of the bone screw 100 after implantation.

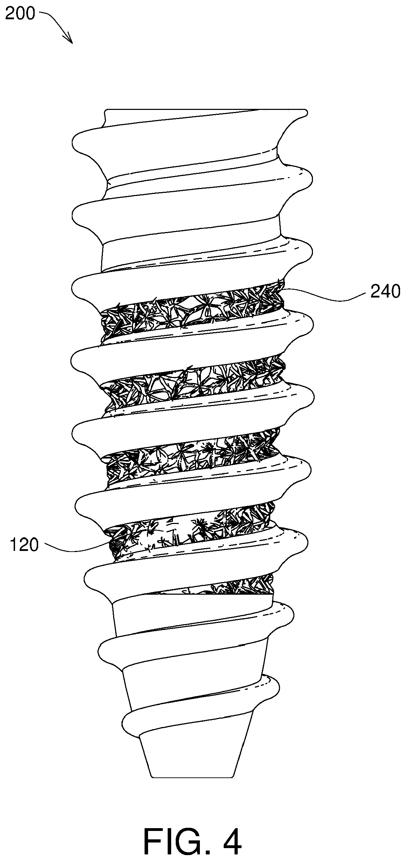

FIG. 4 is a 3D appearance diagram of a bone screw 200 according to another embodiment, which is different from the bone screw 100 of FIG. 1 with the following description. The bone screw 200 includes a porous layer 240. The porous layer 240 is disposed on a surface of the screw thread supporter 120. The porous layer 240 can also be formed on the roots of the external screw thread 110 and connected between the roots. The porous layer 240 has pores connected with each other and has a hardness lower than a hardness of the non-porous material. The porous layer 240 not only can avoid the stress shielding effect but can be used as a bionic porous structure with osseointegration effect, which induces the growth of bone cells and promotes bone ingrowth to achieve osseointegration and fixation and benefit the user from postoperative healing and recovery.

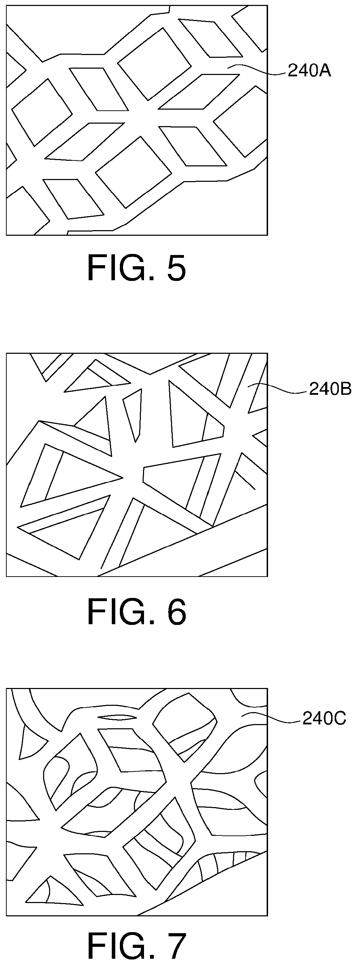

The porosity of the porous layer 240 can be 15% or higher, such as 30% to 60%. The pore size of the porous layer 240 can be 50µm to 500µm, such as 50µm to 200µm, or 300µm to 500µm, or 300µm to 440µm, or 170µm to 440µm, or 170µm to 300µm. In an embodiment, the porous layer 240 can be the porous layer 240A as indicated in FIG. 5 having quadrangle pores whose pore size is 210µm to 390µm. In another embodiment, the porous layer 240 can be the porous layer 240B as indicated in FIG. 6 having triangle pores whose pore size is 310µm to 330µm. In an alternate embodiment, the porous layer 240 can be the porous layer 240C as indicated in FIG. 7 having amoeba-shaped pores whose pore size is 170µm to 440µm. However, the present disclosure is not limited thereto. The porous layer 240 can have other possible types of porous structure.

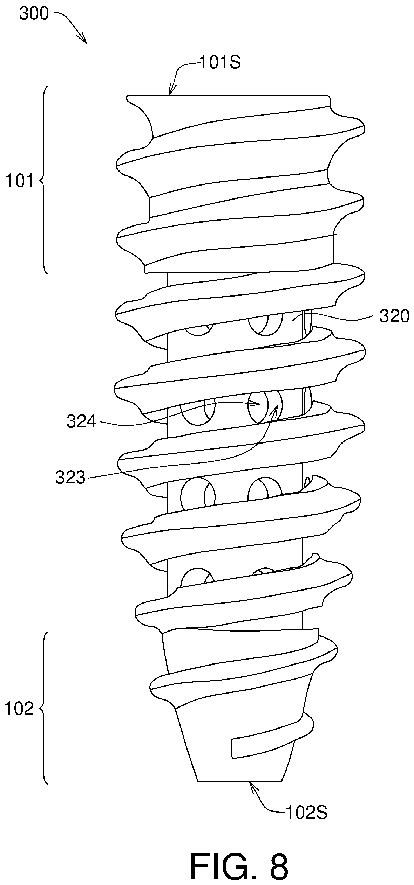

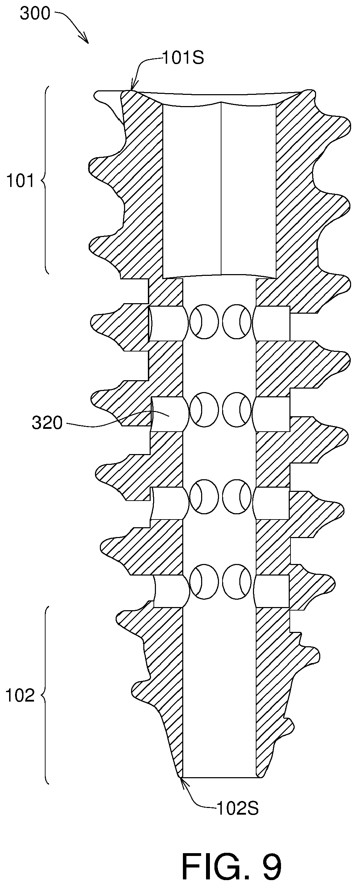

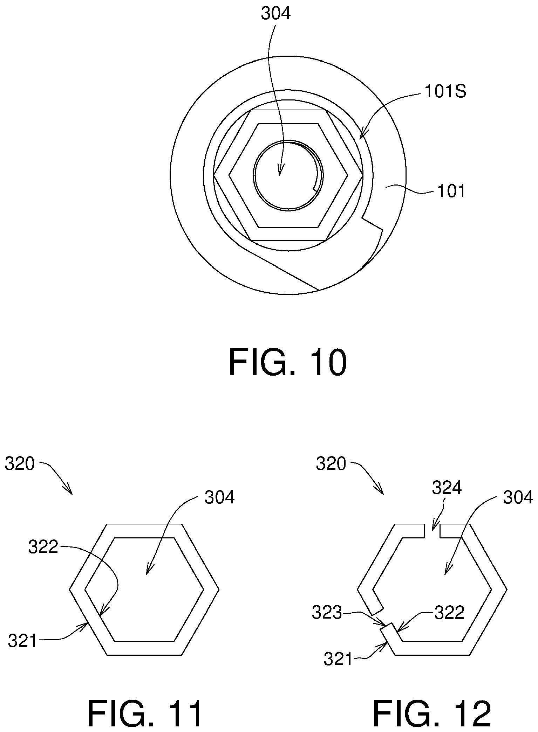

A bone screw 300 in another embodiment is disclosed with reference to FIG. 8 to FIG. 12. FIG. 8 is a 3D appearance diagram of the bone screw 300. FIG. 9 is a longitudinal cross-sectional view of the bone screw 300. FIG. 10 is a top view of the top pin portion 101 of the bone screw 300. The bone screw 300 is different from the bone screw 100 described with referring to FIG. 1 to FIG. 3 with the following description. In this embodiment, the internal supporter structure includes a polygonal wall supporter 320. The polygonal wall supporter 320 can have apertures 324.

FIG. 11 is a lateral cross-sectional view of a portion of the polygonal wall supporter 320 without the apertures 324. The polygonal wall supporter 320 is a hexagonal wall supporter. The outer wall surface 321 of the polygonal wall supporter 320 is a hexagonal wall surface. The inner wall surface 322 of the polygonal wall supporter 320 is a hexagonal wall surface.

FIG. 12 is a lateral cross-sectional view of a portion of the polygonal wall supporter 320 with the apertures 324.

A material of the internal supporter structure (the polygonal wall supporter 320) can have a non-porous structure, which provides the bone screw 300 with sufficient structural strength and hardness, reduces the probability of rupture during the implantation or use process and increases the lifespan of the bone screw 300. In comparison to the bone screw of the comparison example without having an internal supporter structure, the bone screw 300 of the present embodiment having the screw thread supporter 320 having a material being a solid structure (that is, non-porous structure) as an internal supporter structure can have larger torsional strength and pull-out strength.

FIG. 8 to FIG. 12 are referred to. The polygonal all supporter 320 has a middle surface 323. The middle surface 323 is between an outer wall surface 321 and an inner wall surface 322 opposite to the outer wall surface 321. The apertures 324 are defined by the middle surface 323. The apertures 324 are separated from each other by the polygonal wall supporter 320. The bone screw 300 may include a middle hole 304 inside the polygonal wall supporter 320. The middle hole 304 can be extended to the bottom bone screw surface 102S of the bottom pin portion 102 from the top bone screw surface 101S of the top pin portion 101 along an axial direction.

The lateral size, such as width, of the polygonal wall supporter 320 may be 0.9 mm to 2.9mm. The size of the aperture 324 may be 0.5 mm to 1.5mm, such as 1.2mm. But the present disclosure is not limited thereto.

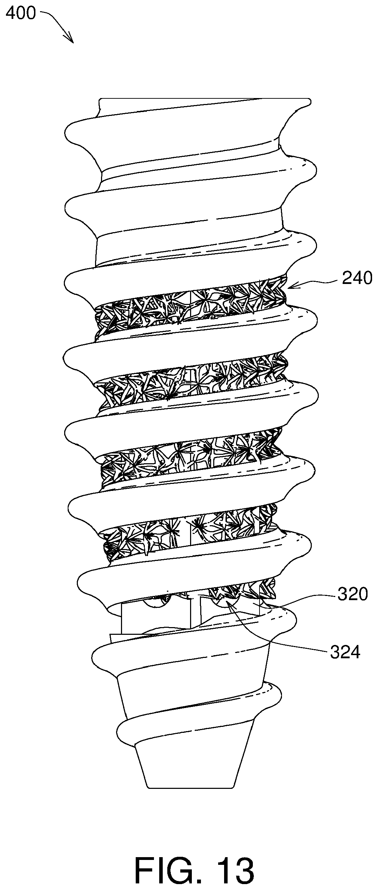

FIG. 13 illustrates a 3D appearance diagram of a bone screw 400 according to yet another embodiment, which is different from the bone screw 300 of FIG. 8 with the following description. The bone screw 400 includes a porous layer 240. The porous layer 240 is on a surface of the polygonal wall supporter 320. The porous layer 240 can be on an outer wall surface of the polygonal wall supporter 320. But the present disclosure is not limited thereto. In an embodiment, the porous layer 240 can be a middle surface of the polygonal wall supporter 320 exposed from the apertures 324. The porous layer 240 can be on an inner wall surface of the polygonal wall supporter 320.

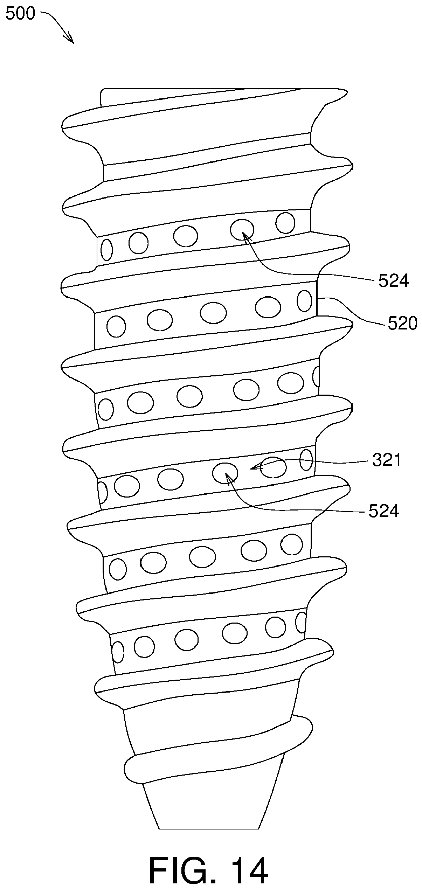

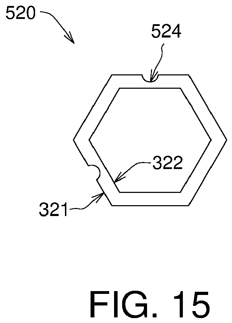

A bone screw 500 in another embodiment is disclosed with reference to FIG. 14 to FIG. 15. FIG. 14 is a 3D appearance diagram of the bone screw 500 according to yet another alternate embodiment, which is different from the bone screw 300 of FIG. 8 with the following description. The bone screw 500 includes a polygonal wall supporter 520 having apertures 524. FIG. 15 is a lateral cross-sectional view of a portion of the polygonal wall supporter 520 with the apertures 524. In the present embodiment, the outer wall surface 321 of the polygonal wall supporter 520 has the apertures 524. The holes 524 can be regarded as pits of the outer wall surface 321. The size of the aperture 524 may be 300µm to 500µm, for example. The polygonal wall supporter 520 having the apertures 524 can be used as a bionic structure which provides osseointegration effect.

A material of the internal supporter structure (the polygonal wall supporter 520) has a solid structure (i.e. non-porous structure), which provides the bone screw 500 with sufficient structural strength and hardness, reduces the probability of rupture during the implantation or use process and increases the lifespan of the bone screw 500. In comparison to the bone screw of the comparison example without having an internal supporter structure, the bone screw 500 of the present embodiment with the screw thread supporter 520 having a material being a solid structure (that is, non-porous structure) as an internal supporter structure can have larger torsional strength and pull-out strength. In an embodiment, a porous layer (such as the porous layer 240, not shown in FIG. 14) can be disposed on a surface of the polygonal wall supporter 520 of the bone screw 500.

The present disclosure is not limited to the above embodiments. For example, the polygonal wall supporter is not limited to a hexagonal shape, and can also have a square shape, a pentagonal shape, a hexagonal shape, a heptagonal shape, an octagonal shape, a nonagon shape, a decagon shape, a hendecagon shape, a dodecagon shape, a thirteen-sided shape, a fourteen-sided shape, etc.

In an embodiment, the bone screw can have a consistent material, such as a metal or a non-metallic material such as high polymer. The bone screw can be integrally formed in one piece. However, the present disclosure is not limited thereto, and the bone screw can be formed by a composite material. The bone screw may be formed by an ordinary method or a laminating method.

It will be apparent to those skilled in the art that various modifications and variations can be made to the disclosed embodiments. It is intended that the specification and examples be considered as exemplary only, with a true scope of the disclosure being indicated by the following claims and their equivalents.

## Claims

1. A bone screw (100, 200, 300, 400, 500), **characterized in that** the bone screw (100, 200, 300, 400, 500) comprises:
an external screw thread (110);
an internal supporter structure (120, 320, 520) disposed inside a middle part of the external screw thread (110), wherein the internal supporter structure (120, 320, 520) comprises one of a screw thread supporter (120, 320) and a polygonal wall supporter (520); and
a porous layer (240, 240A, 240B, 240C) disposed on a surface of the internal supporter structure (120, 320).

2. The bone screw (100, 200, 300, 400, 500) according to claim 1, wherein a spiral direction of the screw thread supporter (120, 320) is identical to a spiral direction of the external screw thread (110).

3. The bone screw (100, 200, 300, 400, 500) according to claim 1, wherein the polygonal wall supporter (520) comprises an outer wall surface (321) having apertures (524).

4. The bone screw (100, 200, 300, 400, 500) according to claim 1, wherein a material of the external screw thread (110) has a non-porous structure.

5. The bone screw (100, 200, 300, 400, 500) according to claim 1, wherein a material of the internal supporter structure (120, 320, 520) has a non-porous structure.

6. The bone screw (100, 200, 300, 400, 500) according to claim 1, further comprising a middle hole (104) passing through an interior of the bone screw (100, 200, 300, 400, 500).

## Amended claims

### Amended claims in accordance with Rule 137(2) EPC.

1. A bone screw (100), **characterized in that** the bone screw (100) comprises:
an external screw thread (110);
an internal supporter structure (120) disposed inside a middle part of the external screw thread (110), wherein the internal supporter structure (120) comprises a screw thread supporter (120), wherein a spiral direction of the screw thread supporter (120) is identical to a spiral direction of the external screw thread (110); and
a porous layer (240, 240A, 240B, 240C) disposed on a surface of the internal supporter structure (120).

2. The bone screw (100) according to claim 1, wherein a material of the external screw thread (110) has a non-porous structure.

3. The bone screw (100) according to claim 1, wherein a material of the internal supporter structure (120) has a non-porous structure.

4. The bone screw (100) according to claim 1, further comprising a middle hole (104) passing through an interior of the bone screw (100).
